(19) Europäisches Patentamt

European Patent Office

Office européen des brevets

(11) **EP 0 709 677 A2**

(12) **EUROPÄISCHE PATENTANMELDUNG**

(43) Veröffentlichungstag:
01.05.1996 Patentblatt 1996/18

(51) Int. Cl.⁶: **G01N 33/533**, G01N 33/547
// G01N33/78

(21) Anmeldenummer: 95116909.3

(22) Anmeldetag: 26.10.1995

(84) Benannte Vertragsstaaten:
**AT BE CH DE DK ES FR GB IE IT LI NL SE**

(30) Priorität: 28.10.1994 DE 4438660

(71) Anmelder: **BOEHRINGER MANNHEIM GMBH**
**D-68305 Mannheim-Waldhof (DE)**

(72) Erfinder:
• **Weindel, Kurt, Dr.**
**D-82392 Habach (DE)**
• **Kraus, Werner, Dr.**
**D-82327 Tutzing (DE)**

(74) Vertreter: **Weiss, Wolfgang, Dr. et al**
**Postfach 860820**
**D-81635 München (DE)**

(54) **Biolumineszenzmarkierte Haptenkonjugate für den Einsatz in kompetitiven Immunoassays**

(57) Die Erfindung betrifft ein Verfahren zum Nachweis eines Analyten in einer Probeflüssigkeit nach dem Prinzip des kompetitiven Immunoassays unter Verwendung von Konjugaten aus einem Hapten und einem Ca-aktivierbaren Photoprotein, ein derartige Konjugate enthaltendes immunologisches Reagenz und neue Hapten-Photoprotein-Konjugate.

EP 0 709 677 A2

**Beschreibung**

Die Erfindung betrifft ein Verfahren zum Nachweis eines Analyten in einer Probeflüssigkeit nach dem Prinzip des kompetitiven Immunoassays unter Verwendung von Konjugaten aus einem Hapten und einem Ca-aktivierbaren Photoprotein, ein derartige Konjugate enthaltendes immunologisches Reagenz und neue Hapten-Photoprotein-Konjugate.

Die Verwendung von Lumineszenzmarkierungen als Nachweissystem bei immunologischen Testverfahren hat gegenüber anderen Nachweissystemen, z.B. radioaktiven Markierungsgruppen, den Vorteil einer höheren Sensitivität. Ein Beispiel für Lumineszenzmarkierungen, die sich für den Einsatz in Immunoassays eignen, sind Ca-aktivierbare Photoproteine der Aequorinfamilie.

Aequorin ist ein Photoprotein bestehend aus Apo-Aequorin, der katalytisch aktiven Proteinkomponente, Coelenterazin, der oxidierbaren Luminophorkomponente sowie molekularem, wahrscheinlich als Hydroperoxid proteingebundenem Sauerstoff, dem Oxidationsmittel. Durch Zugabe von $Ca^{2+}$-Ionen (Trigger) kommt es zu einer Lumineszenzreaktion zwischen den einzelnen Komponenten des Reaktionskomplexes. Die Aequorin-Biolumineszenz zeichnet sich aus durch außergewöhnlich hohe Photonenausbeute ($\geqq$ 25%) und Schnelligkeit (< 5 sec.) (vgl. z.B. Blinks, J. Pharmacological Reviews 28 (19..), 1-93).

Ein Nachteil von Aequorin und verwandten Photoproteinen ist jedoch, daß sie äußerst empfindlich gegenüber jeglicher chemischer Modifikation sind. Die Folgen sind üblicherweise Verlust der katalytischen Aktivität und/oder vorzeitige Entladung ohne Triggerzugabe.

WO 94/18342 beschreibt eine schonende Modifizierung von Aequorin durch Einführung zusätzlicher SH-Gruppen. An diese SH-Gruppen können über geeignete reaktive Gruppen, z.B. Maleimidgruppen, Proteine oder Haptene gekoppelt werden. Auf diese Weise ist die Herstellung von Protein-Aequorin-Konjugaten unter hohem Erhalt an Photoprotein-Aktivität möglich.

Es hat sich jedoch gezeigt, daß die Konjugate des Standes der Technik erhebliche Nachteile aufweisen. So können sie in der Praxis bei immunologischen Testverfahren nach dem Prinzip des kompetitiven Immunoassay, wo ein festphasengebundenes oder markiertes Hapten mit dem Analyten um einen Rezeptor konkurriert, nur bei Tests im "labelled antibody"-Format eingesetzt werden. In diesem Testformat konkurrieren ein immobilisiertes Hapten und der in der Probeflüssigkeit vorhandene freie Analyt um eine limitierte Anzahl von Bindestellen an einem markierten Rezeptor, z.B. einem Antikörper-Aequorin-Konjugat. Bei einem derartigen Testformat ist das wandgebundene Signal umgekehrt proportional zur Konzentration des freien Analyten.

Für viele niedermolekulare Analyten (Haptene) ist es jedoch wünschenswert, einen kompetitiven Immunoassay im "labelled analog"-Format durchzuführen, d.h. den Rezeptor, z.B. einen Antikörper, an die Festphase zu immobilisieren und dann die Probeflüssigkeit mit dem freien Analyten und einen Unterschuß von markiertem Analyten (Tracer) um limitierte Antikörper-Bindestellen konkurrieren zu lassen. Diese Testführung entspricht dem klassischen Radioimmunoassay. Die Menge an festphasengebundenem, markiertem Analyten ist dann zu der Konzentration an freiem, zu bestimmendem Analyten umgekehrt proportional.

Mit den aus dem Stand der Technik bekannten Hapten-Aequorin-Konjugaten ist die Durchführung eines kompetitiven Immunoassays im "labelled analog"-Format nur mit Einschränkung möglich, weil sie eine zu geringe Sensivität und Präzision aufweisen. Weiterhin ist die Herstellung der Konjugate aufgrund der Einführung zusätzlicher SH-Gruppen kompliziert und nicht ausreichend reproduzierbar.

In der Publikation Stults et al. (Biochemistry 31 (1992), 1433-1442) ist die Kopplung eines Biotin-N-hydroxy-succinimid-Esters an freie Aminogruppen von Aequorin beschrieben. Das resultierende Konjugat besitzt eine hohe Photoproteinaktivität. Zur Immunreaktivität dieses Konjugats finden sich jedoch keine Angaben. Das Biotin-Aequorin-Konjugat kann als Universal-Konjugat in einem immunologischen Test verwendet werden, bei dem es in einem hohen Überschuß zugesetzt wird. Es kann jedoch nicht als testspezifischer Tracer in einem kompetitiven Test nach dem "labelled analog"-Format eingesetzt werden, wo die Zugabe einer genau limitierten Menge im Unterschuß erforderlich ist.

Erfahrungen mit anderen Lumineszenzsystemen, z.B. Acridiniumestern, zeigen, daß die Herstellung von geeigneten Antikörper-Konjugaten keine Gewähr dafür ist, daß auch aktive Tracer-Konjugate für kompetitive Immunoassays herstellbar sind. So sind bei diesem Lumineszenzsystem sensitive kompetitive Testverfahren ausschließlich in "labelled antibody"-Formaten beschrieben (Weeks I. et al., Clinical Chemistry 29 (1983), p. 1474-1479; Sturgess M. et al., Clinical Chemistry 32 (1986), p. 532-535; Sturgess M. et al., Clinical Endocrinology 27 (1987), p. 383-393; McCapra F. et al., Journal of Bioluminescence and Chemiluminescence 4 (1989), 51-58). Die Kopplung von identisch oder ähnlich funktionalisierten Acridiniumestern an Haptenen führt hingegen zu erheblichen Problemen bezüglich unspezifischer Bindung und zu einer Beeinträchtigung der Präzision und der Sensitivität.

Die der vorliegenden Erfindung zugrundeliegende Aufgabe bestand darin, Aequorin auf möglichst einfache Weise schonend an ein Hapten zu koppeln und dabei Tracer-Konjugate für kompetitive Immunoassays nach dem "labelled analog"-Format zu erhalten, die sowohl eine hohe Immunoreaktivität als auch eine hohe Photoproteinaktivität aufweisen, so daß eine für praktische Anwendungen ausreichend hohe analytische Testsensitivität erzielt wird.

Diese Aufgabe wurde gelöst durch ein Verfahren zum Nachweis eines Analyten in einer Probeflüssigkeit nach dem Prinzip des kompetitiven Immunoassay, wobei man

(a) die Probeflüssigkeit in Gegenwart einer reaktiven Festphase mit (i) einem Konjugat aus mindestens einem mit dem Analyten immunologisch konkurrierenden Hapten und einem Ca-aktivierbaren Photoprotein und (ii) einem Rezeptor inkubiert, wobei das Hapten an eine Aminogruppe des Photoproteins gekoppelt ist, und der Rezeptor an die Festphase gebunden oder zur Bindung an die Festphase fähig ist und eine spezifische immunologische Reaktion mit dem Analyten und dem Hapten eingehen kann,

(b) die Festphase von der Inkubationsflüssigkeit abtrennt und

(c) das Vorhandensein oder/und die Menge des Analyten in der Probeflüssigkeit durch Lumineszenzmessung in der Festphase oder/und in der Inkubationsflüssigkeit bestimmt.

Überraschenderweise wurde festgestellt, daß die Konjugate aus Hapten und Ca-aktivierbarem Photoprotein in kompetitiven Immunoassays bezüglich Sensitivität, Präzision und Dynamik mindestens so gut wie herkömmliche, bereits automatisierte und optimierte Enzymun-Testsysteme sind. Weiterhin sind die erfindungsgemäßen Konjugate, bei denen das Hapten an eine Aminogruppe des Photoproteins gekoppelt ist, den aus dem Stand der Technik bekannten Konjugaten, die durch Einführung zusätzlicher Thiolgruppen und anschließende Kopplung mit Maleimid hergestellt werden, deutlich überlegen. Dies ist darauf zurückzuführen, daß die erfindungsgemäßen Konjugate überraschenderweise gleichzeitig eine hohe immunologische Aktivität und eine hohe Photoaktivität aufweisen. Das erfindungsgemäße Testformat eignet sich somit vorzüglich zum Nachweis von Analyten, insbesondere von Hapten-Analyten in einem kompetitiven "labelled analog"-Testformat und kann die bisher auf diesem Gebiet noch dominanten radioaktiven Immunoassays ersetzen.

Die Kopplung des Haptens an eine Aminogruppe des aktivierbaren Photoproteins erfolgt vorzugsweise dadurch, daß das Hapten über eine aktivierte Carbonsäurefunktion, z.B. als Aktivester an das Photoprotein gekoppelt wird. Beispiele für geeignete Aktivester sind p-Nitrophenyl-, Pentafluorphenyl-, Imidazolyl-, N-Hydroxybenzotriazolyl- und N-Hydroxysuccinimidester. Besonders bevorzugt sind N-Hydroxysuccinimidester. Bei der Herstellung des Konjugats erfolgt die Kopplung des Haptens an das Photoprotein in einem molaren Verhältnis von vorzugsweise 1:1 bis 6:1.

Beim erfindungsgemäßen Verfahren werden Konjugate aus Haptenen und Ca-aktivierbaren Photoproteinen, vorzugsweise Ca-aktivierbaren Photoproteinen der Aequorinfamilie verwendet. Beispiele für solche Proteine sind Aequorin, Obelin, Clytin, Mitrocomin, Berovin und Mnemiopsin. Vorzugsweise wird Aequorin verwendet.

Die Ca-aktivierbaren Photoproteine können als native Proteine, die aus ihren Ursprungsorganismen isoliert wurden, oder als rekombinante Proteine, z.B. aus E. coli, eingesetzt werden. Die Verwendung rekombinanter Photoproteine ist bevorzugt. Aequorin stammt beispielsweise aus dem Organismus Aequorea victoria. Die rekombinante Herstellung von Aequorin in E. coli ist von Stults et al. (Biochemistry 31 (1992) 1433-1442) beschrieben. Die Isolierung und Reinigung des Photoproteins Obelin aus dem Organismus Obelia longissima ist von Vysotskii et al. (Biokhimiya 54 (1989) 965-973) beschrieben. Die Klonierung, Expression und Sequenz von Obelin sind von Illarionov et al. (J. Biolumineszenz und Chemolumineszenz 8 (1993), VII. International Symposium-Abstracts, 88) beschrieben. Die Klonierung, Expression und Sequenzanalyse der Photoproteine Clytin und Mitrocomin sind bei Inouye und Tsuji (FEBS 315 (1993) 343-346) und Fagan et al. (FEBS 333 (1993), 301-305) beschrieben. Weitere Mitglieder der Aequorinfamilie sind die Photoproteine Berovin und Mnemiopsin (Ward und Seliger, Biochemistry 13 (1974) 1491-1499 und 1500-1510). Auf die in den oben genannten Literaturstellen enthaltene Offenbarung zur Gewinnung von Ca-aktivierbaren Photoproteinen wird hiermit Bezug genommen.

Das erfindungsgemäße Verfahren dient zum Nachweis eines Analyten in einer Probeflüssigkeit. Der Begriff Analyt umfaßt immunologisch nachweisbare biologische Substanzen, die in einer Probeflüssigkeit, z.B. einer Körperflüssigkeit wie etwa Serum, Plasma, Urin, Darmflüssigkeit etc. qualitativ oder/und quantitativ bestimmt werden soll. Beispiele für Analyten sind Proteine, Glycoproteine, Polypeptide, Peptide, Mono-, Oligo- und Polysaccharide, Hormone, Neurotransmitter, Mediatoren, Metaboliten, Vitamine, pharmakologische Wirkstoffe, Nukleinsäuren und andere biologische, durch eine spezifische Reaktion mit einem geeigneten Rezeptor nachweisbare Analyten. Bevorzugte Analyten sind niedermolekulare Moleküle, z.B. Hormone wie etwa Steroide, z.B. Sexualhormone und Glucocorticoide, Peptidhormone, Schilddrüsenhormone, Mediatoren wie etwa Histamine, Eicosanoide wie etwa Prostaglandine, Thromboxane, Leuco-En-diine und Lipoxine, Neurotransmitter wie etwa γ-Aminobuttersäure, Serotonin, Adrenalin und Noradrenalin, pharmakoligsche Wirkstoffe, z.B. Opiate, Metaboliten, z.B. Creatinin und Vitamine.

Die Haptenkomponente des Hapten-Photoprotein-Konjugats wird so gewählt, daß sie mit dem jeweils zu bestimmenden Analyten immunologisch konkurriert, d.h. daß das Hapten mit einem für den Analyten spezifischen Rezeptor ebenfalls reagiert. Zu diesem Zweck ist das Hapten, bei dem es sich im allgemeinen um eine niedermolekulare organische Verbindung mit einer Molekularmasse von vorzugsweise ≤ 2.000 D handelt, entweder der nachzuweisende Analyt selbst, ein immunologisches Analogon des Analyten oder ein immunologisch aktiver Bereich (Epitop) des Analyten.

Der Rezeptor, der zum Nachweis des Analyten eingesetzt wird, ist entweder ein festphasengebundener Rezeptor oder ein zur Bindung an eine Festphase fähiger Rezeptor, z.B. ein biotinylierter Rezeptor, der an eine mit Streptavidin beschichtete Festphase binden kann. Weiterhin muß der Rezeptor, wenn er zum Einsatz in einem kompetitiven Immunoassay des "labelled analog" Formats geeignet sein soll, eine spezifische immunologische Reaktion mit dem Analyten und dem Hapten eingehen. Beispiele für geeignete Rezeptoren sind polyklonale Antikörper, monoklonale Antikörper, Antikörperfragmente oder andere Substanzen, die spezifisch mit dem jeweils zu bestimmenden Analyten reagieren können. Wenn der Analyt beispielsweise eine Nukleinsäure ist, so kann der Rezeptor beispielsweise eine komplementäre Nukleinsäure sein, die unter den jeweiligen Testbedingungen spezifisch mit dem Analyten und dem Hapten, das in diesem Falle ebenfalls eine Nukleinsäure ist, hybridisiert.

Die Haptenkomponente des Konjugats ist vorzugsweise über einen Spacer an das Photoprotein gekoppelt. Die Länge dieses Spacers kann je nach verwendetem Hapten variabel sein, sie beträgt vorzugsweise jedoch 2-8 Atome. Der Spacer umfaßt vorzugsweise eine lineare, gegebenenfalls durch Heteroatome wie etwa O oder N substituierte Alkylenkette. Durch Verwendung eines Spacers können sterische Hinderungen bei der Wechselwirkung von Hapten-Aequorin-Konjugat und Antikörper reduziert werden, ebenso wie Verluste an Photoproteinaktivität bei der Kopplung, so daß das Niveau an gebundenem Signal erhöht wird. Dadurch wird der Impräzisionsbeitrag durch Untergrundrauschen des Meßgerätes oder elektronische Störungen aus der Umgebung kleiner.

Die immunologische Aktivität und die Photoaktivität des Konjugats sind für einen kompetitiven Immunoassay mit sehr hohen Leistungsanforderungen ausreichend. Daher kann die Inkubation des Konjugats und des Rezeptors mit der den Analyten enthaltenden Probeflüssigkeit als 1-Schritt-Simultaninkubation mit sehr geringen Inkubationszeiten und Reaktionsvolumina durchgeführt werden. Die Inkubation beträgt vorzugsweise maximal 45 min und besonders bevorzugt 10-30 min. Das Inkubationsvolumen beträgt vorzugsweise maximal 500 µl und besonders bevorzugt 20-200 µl.

Weiterhin betrifft die vorliegende Erfindung die Verwendung eines Konjugats aus mindestens einem Hapten und einem Ca-aktivierbaren Photoprotein, worin das Hapten an eine Aminogruppe des Photoproteins gekoppelt ist, als Analytanalogon in einem kompetitiven Immunoassay des "labelled analog" Formats.

Noch ein weiterer Gegenstand der vorliegenden Erfindung ist ein Reagenz zum Nachweis eines Analyten nach dem Prinzip des kompetitiven Immunoassay, welches ein Konjugat aus mindestens einem mit den Analyten immunologisch konkurrierenden Hapten und einem Ca-aktivierbaren Photoprotein enthält, worin das Hapten an eine Aminogruppe des Photoproteins gekoppelt ist. Noch ein weiterer Gegenstand der vorliegenden Erfindung ist ein Reagenzienkit, der das obengenannte Reagenz und räumlich getrennt davon einen Rezeptor enthält, der an eine Festphase gebunden oder zur Bindung an eine Festphase fähig ist und eine spezifische immunologische Reaktion mit dem Analyten und dem Hapten eingehen kann.

Die restliche Lumineszenzaktivität des Photoproteins im Hapten-Photoprotein-Konjugat beträgt vorzugsweise mindestens 30%, besonders bevorzugt mindestens 40% und am meisten bevorzugt mindestens 50% der Ausgangsaktivität eines entsprechenden nicht-modifizierten Photoproteins.

Schließlich betrifft die vorliegende Erfindung auch neue Konjugate aus mindestens einem Hapten und einem Ca-aktivierbaren Photoprotein, worin das Hapten über einen Spacer mit einer Kettenlänge von 2-8 Atomen an eine Aminogruppe des Photoproteins gekoppelt ist. Vorzugsweise ist das Hapten über eine Carbonsäurefunktion an das Photoprotein gekoppelt. Bei der Herstellung des Konjugats erfolgt die Kopplung an das Photoprotein vorzugsweise in einem molaren Verhältnis von 1:1 bis 6:1.

Weiterhin wird die vorliegende Erfindung durch die nachfolgenden Beispiele erläutert:

Beispiel 1

Herstellung eines immunreaktiven Thyroxin-(T4)-Konjugats mit unmodifiziertem Aequorin

Material:

Rekombinantes Aequorin wurde von der Fa. SeaLite Sciences, Atlanta, USA, bezogen. Das aktivierte Hapten T4 (OMe)-N-hs-0Su (Thyroxin-O-methyl-N-hemisuccinyl-hydroxysuccinimidester) wurde von Boehringer Mannheim GmbH, alle anderen Chemikalien von Merck, Darmstadt, bezogen. Die Lumineszenz wurde in einem AutoLumat LB 953 der Firma Berthold München GmbH gemessen.

Konjugation:

Um die Photoaktivität des Aequorins bei der Konjugation weitestgehend zu erhalten, wurde das durch Einführung eines Hydroxysuccinimidesters aktivierte T4 durch Umamidierung an die Aminogruppen des unmodifizierten Aequorins gekoppelt. Verwendet wurde das aktivierte Hapten T4 (OMe)-N-hs-0Su, das sich auf Grund des eingeführten Hemisuccinat-$C_4$-Spacers als besonders geeignet erwies.

Das zu derivatisierende Aequorin wurde in 0,1 mol/l NaCarbonat-Puffer, pH 8,6, 0,15 mol/l NaCl, 2 mmol/l EDTA auf eine Konzentration von 4 mg/ml eingestellt und auf 25°C temperiert. Zu 1 ml der Lösung wurden 50 µl T4(OMe)-N-hs-OSu in DMS0 (Di-methylsulfoxid) (Konz.: 7,2 mg/ml) zugesetzt. Dies entspricht einem molaren Verhältnis von T4:Aequorin = 2:1 und einer Konzentration von 5% im Ansatz.

Das Reaktionsgemisch wurde unter Rühren 90 min bei 25°C inkubiert, und anschließend wurde die Reaktion durch Zugabe von 10 µl einer 1 mol/l Lysinlösung (10 mmol/l Endkonz.) gestoppt. Der Ansatz wurde weitere 10 min gerührt.

Zur Entfernung nicht gekoppelten Haptens wurde der Ansatz über Nacht fließend gegen das 1000fache Volumen an 0,01 mol/l HEPES, pH 7,5, 0,2 mol/l NaCl, 0,01 mol/l Ethylenglykol-bis-($\beta$-aminoethylether)-N,N,N'N-tetraessigsäure (EGTA) dialysiert. Das Konjugat wurde anschließend mit 0,05% Tween 20 und 0,05% $NaN_3$ stabilisiert und bei -70°C gelagert.

Bei der angegebenen Stöchiometrie wurden 0,5 Mol T4/1 Mol Aequorin eingebaut (Bestimmung mit Enzymun-Test®T4, Boehringer Mannheim GmbH), und das Aequorin zeigte noch 50% der Ausgangs-Photoaktivität.

Beispiel 2

Biolumineszenz-Immunoassay für die Bestimmung von Gesamt-Thyroxin

Mit einer gemäß Beispiel 1 hergestellten Tracer-Präparation, welche ca. 0,6 Mol Thyroxin pro Mol Aequorin enthielt und bei der eine 4 C-Atom-Kette als Spacer zwischen Hapten und Aequorin diente, wurde ein Biolumineszenz-Immunoassay mit 15 min Inkubation bei Raumtemperatur, ohne Schütteln, 1-Schritt-Simultaninkubation, 40 $\mu$l Gesamtvolumen und 2 sec Meßzeit nach Trennung von gebundener und nicht-gebundener Fraktion durchgeführt. Der Nullkalibrator wurde in Quadruplikat-, alle anderen Kalibratoren in Duplikatmessungen bestimmt.

Als Testträger dienten miniaturisierte Rundboden-Reaktionsgefäße, die mit Streptavidin beschichtet waren.

Der Testpuffer bestand aus 120 mmol/l Barbital-Natrium, pH 8,7, 5 mmol/l EDTA, 0,04% Anilinonaphthalinsulfonsäure (ANS), und 0,2 % (w/v) Rinderserumalburin (RSA); die Waschlösung aus 10 mmol/l Tris, pH 7,4, 2 mmol/l EDTA, und 0,5% (v/v) Tween-20.

Zum Test wurde der Tracer in einer Konzentration eingesetzt, die einer Immunoreaktivität von 17 nmol/l = 1/20 des höchsten Kalibrators entspricht (ermittelt via automatisiertem Enzymun-Test®). Ein polyklonaler ⟨T4⟩-Antikörper (biotinyliert) wurde in einer Konzentration von 330 ng/ml zugesetzt. Das eingesetzte Volumenverhältnis von Probe:Tracer:Testpuffer betrug 1:1:48. Die Kalibratoren wurden selbst hergestellt durch Einwaage von Thyroxin in eine Matrix aus RSA und Thyroxin bindendem Globulin (TBG) und entsprechende Verdünnungen. Der Abgleich und die Zuweisung der Werte erfolgte anhand eines entsprechenden automatisierten Enzymun-Tests®T4 auf dem Gerät ES 300 (Boehringer Mannheim GmbH). Die TBG-Werte wurden kontrolliert anhand des Enzymun-Tests®TBG (Boehringer Mannheim GmbH).

Ergebnisse:

| Kalibrator | gebundenes Signal [RLU / 2 sec] | Faktor A/X |
|---|---|---|
| A = 0,0 $\mu$g/dl | 91495 | 1,00 |
| B = 4,1 $\mu$g/dl | 47954 | 1,91 |
| C = 7,7 $\mu$g/dl | 35597 | 2,57 |
| D = 14,0 $\mu$g/dl | 23039 | 3,97 |
| E = 25,5 $\mu$g/dl | 10394 | 8,80 |

Untere Nachweisgrenze, berechnet auf Basis Kalibrator A - 2 SD via lineare Regression = 0,50 $\mu$g/dl.

Beispiel 3

Biolumineszenz-Immunoassay für die Bestimmung von Gesamt-Thyroxin

Die Durchführung erfolgte analog Beispiel 1, abgesehen davon, daß eine Tracer-Präparation mit 0,3 Mol Thyroxin pro Mol Aequorin und ein neues Kalibrator-Set verwendet wurden. Die Meßzeit betrug 1 sec.

Ergebnisse:

| Kalibrator | gebundenes Signal [RLU / sec] | Faktor A/X |
|---|---|---|
| A = 0,0 µg/dl | 62509 | 1,00 |
| B = 3,3 µg/dl | 37088 | 1,69 |
| C = 7,1 µg/dl | 26057 | 2,40 |
| D = 13,1 µg/dl | 18610 | 3,36 |
| E = 26,3 µg/dl | 10814 | 5,78 |

Untere Nachweisgrenze, berechnet auf Basis Kalibrator A - 2 SD via lineare Regression = 0,45 µg/dl.

In beiden Fällen konnte eine Eichkurvendynamik (A/X) sowie eine Eichkurvensensitivität erreicht werden, die dem Niveau kommerzieller ELISAs entspricht, aber in stark verkürzter Testdauer, minimiertem Volumen, drastisch vereinfachter Signalgenerierung/-detektion, und ebenfalls in einfachem 1-Schritt-Format.

Beispiel 4

Methodenvergleich Biolumineszenz-Immunoassay (manuell) vs. Enzymun-Test® (automatisiert an ES 300) für T4

Insgesamt 20 Seren, 10 davon mit erhöhtem TBG-Gehalt (bis 42 µg/ml) wurden mit oben beschriebenem Biolumineszenz-Immunoassay (BLIA) bzw. dem Enzymun-Test®T4 vermessen; ersterer voll manuell, letzterer auf dem automatischen Analyser ES 300.

Die Standard-Hauptkomponenten-Analyse ergab die folgende Regressionsgerade:

BLIA (Y) = 1,02 x Enzymun (X) + 1,04 ; r = 0,959; n = 20. Die Seren überspannten dabei den gesamten klinisch relevanten Konzentrationsbereich, 2,5 - 20 µg/dl Thyroxin.

Diese hervorragende Korrelation konnte in einem weiteren Methodenvergleich bestätigt werden.

Beispiel 5

Biolumineszenzimmunoassay für die Bestimmung von freiem Thyroxin

Mit der Tracer-Präparation von Beispiel 3 wurde ein Biolumineszenz-Immunoassay mit 15 min Inkubation bei Raumtemperatur, ohne Schütteln, 1-Schritt-Simultaninkubation, 40 µl Gesamtvolumen, und 1 sec Meßzeit nach Trennung von gebundener und nicht-gebundener Fraktion durchgeführt. Der Nullkalibrator wurde in Quadruplikat-, alle anderen Kalibratoren in Duplikatmessungen bestimmt.

Als Testträger dienten miniaturisierte Rundboden- Reaktionsgefäße, die mit Streptavidin beschichtet wurden.

Der Testpuffer bestand aus 1O0 mmol/l Na-Phosphat, pH 6.3, 10 mmol/l EDTA, 0.05% Humanserumalbumin, 0.002% unspezifisches Rinder-IgG und 0.05% Triton X-1O0; die Waschlösung aus 10 mmol/l Tris, pH 7.4, 2 mmol/l EDTA, und 0.5% (v/v) Tween-20.

Zum Test wurde der Tracer in einer Konzentration eingesetzt, die einer Immunoreaktivität von 21.6 pmol/l = 1.68 ng/dl ≈ 1/5 des höchsten Kalibrators entspricht (ermittelt via Enzymun-Test®fT4). Ein polyklonaler ⟨T4⟩-Antikörper (biotinyliert) wurde in einer Konzentration von 100 ng/ml zugesetzt. Das eingesetzte Volumenverhaltnis von Probe: Reagenz betrug 1:2.

Die Kalibratoren waren dieselben wie für Gesamt-Thyroxin, nur erfolgte diesmal keine Ablösung. Der Abgleich und die Zuweisung der Werte erfolgte anhand eines entsprechenden automatisierten Enzymun-Tests®fT4 auf dem ES 300 Gerät.

Ergebnisse:

| Kalibrator | gebundenes Signal [RLU / 1 sec] | Faktor A/X |
|---|---|---|
| A = 0,0 ng/dl | 4182 | 1,00 |
| B = 0,4 ng/dl | 2908 | 1,44 |
| C = 0,9 ng/dl | 2389 | 1,75 |
| D = 2,1 ng/dl | 1513 | 2,77 |
| E = 6,4 ng/dl | 744 | 5,62 |

Untere Nachweisgrenze, berechnet auf Basis Kalibrator A - 2 SD via lineare Regression = 0,27 ng/dl.

Beispiel 6

Biolumineszenzimmunoassay fur die Bestimmung von freiem Thyroxin

Die Durchführung erfolgte analog Beispiel 5. Es wurden ein Tracer mit 0,5 Mol Thyroxin / Mol Aequorin und 200 ng/ml polyklonaler ⟨T4⟩-Antikörper (biotinyliert) verwendet.

Ergebnisse:

| Kalibrator | gebundenes Signal [RLU / 1 sec] | Faktor A/X |
|---|---|---|
| A = 0,0 ng/dl | 3931 | 1,00 |
| B = 0,4 ng/dl | 2388 | 1,65 |
| C = 0,9 ng/dl | 1762 | 2,23 |
| D = 2,1 ng/dl | 1107 | 3,55 |
| E = 6,4 ng/dl | 762 | 5,16 |

Untere Nachweisgrenze, berechnet auf Basis Kalibrator A - 2 SD via lineare Regression = 0,14 ng/dl.

Somit wurden auch für den fT4-Test Eichkurven erhalten, die trotz der erhöhten Anforderungen an Empfindlichkeit (ng/dl statt µg/dl) mit minimierter Testzeit und minimiertem Testvolumen wiederum mindestens ELISA-Niveau erreichten.

Grundlage dafür ist, daß die Aequorin-Tracer in einem manuellen, kinetisch kontrollierten immunologischen Test auch eine hervorragende Präzision erlaubten. Die withinassay variance, berechnet aus Doppelbestimmungen für die gesamte Eichkurve gemäß

$$CV = \sqrt{(\Sigma\, d^2 / 2n)};$$

$$\text{wobei } d = [\frac{\text{höherer Wert der Doppelbestimmung}}{\text{niedriger Wert der Doppelbestimmung}} -1\,] \times 100$$

und n = Anzahl der Messungen

nach der Methode von Abraham G. et al. (Journal of Clinical Endocrinology, 32, 619-624, 1971) betrug nur 5 - 10%.

Beispiel 7

Methodenvergleiche Biolumineszenz-Immunoassay (manuell) vs. Enzymun-Test®(automatisch) für fT4

Sowohl mit dem oben beschriebenen 1-Schritt-Format, als auch mit einem 2-Schritt delayed solid phase-Format (DESP) wurden Methodenvergleiche zu Enzymun® durchgeführt.

Die Probenseren wiesen, mit Enzymun-Test®fT4 als Referenz vermessen, fT4-Werte im Bereich 0,32 - 6,70 ng/dl auf, und überspannten also den gesamten klinisch relevanten Konzentrationsbereich. Beim DESP-Format wurde die Reaktionsmischung zuerst in Abwesenheit einer Streptavidin-Festphase in Eppendorf-Gefäßen für 8 min unter leichtem Schütteln inkubiert. Dann wurde die Reaktionslösung in Streptavidin-beschichtete miniaturisierte Reaktionsgefäße (s.o.) transferiert und für weitere 7 min ohne Schütteln inkubiert. Auf diese Weise wurde eine Immobilisierung der im 1. Schitt in Lösung gebildeten Immunkomplexe erreicht.

Eine Standard-Hauptkomponenten-Analyse ergab die folgende Regressionsgeraden für den Biolumiszenz-Immunoassay (BLIA):

- 2-Schritt-DESP-Format, 100 ng/ml Antikörper:

**BLIA (Y) = 1,08 x Enzymun (X) + 0.24; r = 0,979; n = 6**

- 1-Schritt-Simultan-Format, 100 ng/ml Antikörper:

**BLIA (Y) = 0,924 x Enzymun (X) - O,007; r = O,998; n = 6**
Diese hervorragende Korrelation konnte in einem weiteren Methodenvergleich bestätigt werden.

Somit erwiesen sich Hapten-Aequorin-Tracer auch bezüglich funktioneller Sensitivität als sehr leistungsfähig.

Beispiel 8

Vergleich verschiedener Hapten-Photoprotein-Konjugate

Es wurde die in Beispiel 1 hergestellte Tracer-Präparation, in der das Hapten über einen Spacer mit einer Kettenlänge von 4 Atomen an das Photoprotein gekoppelt war, mit einer Tracer-Präparation, in der das Hapten ohne Spacer an eine freie $NH_2$-Gruppe des Photoproteins gekoppelt war, und mit einer weiteren Tracer-Präparation, in der das Hapten über einen C9-Spacer an eine zusätzlich in das Photoprotein eingeführte SH-Gruppe gekoppelt war, verglichen.

Die Herstellung der ohne Spacer an die Aminogruppe von Aequorin gekoppelten Tracer-Präparation erfolgte analog Beispiel 1, außer daß der Hemisuccinat-Spacer weggelassen wurde.

Die Herstellung des Tracers mit dem an eine Thiolgruppe von Aequorin gekoppelten Hapten erfolgte durch Umsetzung von Aequorin mit 2-Iminothiolan (Sigma Chemical Co.), wodurch zusätzliche Thiolgruppen eingeführt wurden, und anschließende Kopplung mit dem aktivierten Hapten T4-Hexanoylmaleinimid.

Es wurden noch weitere Möglichkeiten der Kopplung von Aequorin an biologische Substanzen untersucht. Hierzu wurden IgG-Aequorin-Konjugate hergestellt, wobei das IgG-Molekül über Glutardialdehyd, Disuccinimidylsuberat und N-succinimidyl-3-(2-pyridyl-dithio)-propionat an das Hapten gekoppelt wurde. Die so erhaltenen Konjugate waren jedoch aufgrund einer viel zu geringen Photoproteinaktivität für den Einsatz in Immuno-Assays völlig ungeeignet.

Die drei oben genannten Hapten-Aequorin-Konjugate wurden in einem fT4-Test unter jeweils identischen Testbedingungen (15 min Inkubation bei Raumtemperatur, 1-Schritt-Simultaninkubation, 40 µl Gesamtvolumen, Zusatz von jeweils 200 ng/ml biotinyliertem polyklonalen Anti-Thyroxin-Antiserum, 432 pmol/l Tracer) getestet. Die Meßdaten sind in RLU/sec ± SD angegeben.

| Konjugat | Kopplung an SH-Gruppe | Kopplung an $NH_2$-Gruppe mit C4-Spacer | Kopplung an $NH_2$-Gruppe ohne Spacer |
|---|---|---|---|
| Null-Kalibrator | 919 ± 59 | 1310 ± 66 | 952 ± 27 |
| höchster Kalibrator | 88 ± 30 | 173 ± 22 | 166 ± 21 |

Bei Verwendung von Konjugaten, in denen das Hapten via 4C-Spacer an eine $NH_2$-Gruppe des unmodifizierten Aequorins gekoppelt ist, wurden bessere Ergebnisse erhalten als bei Konjugaten, bei denen das Hapten an eine SH-Gruppe gekoppelt ist. Dies zeigte sich durch höhere Werte beim Null-Kalibrator und beim höchsten Kalibrator. Aufgrund der höheren Werte an gebundenem Signal wird der Einfluß von Untergrundschwankungen aus der Photonenstatistik sowie elektronischer Störungen aus der Umgebung stark vermindert, wodurch man eine Verbesserung der Testpräzision erreicht (5,1% vs. 6,4% VK beim Nullkalibrator bzw. 12,7% vs. 33,7% beim höchsten Kalibrator jeweils zugunsten der NHS-Direktkopplung).

Der Vergleich NHS-Direktkopplung an eine $NH_2$-Gruppe des unmodifizierten Aequorins mit und ohne Spacer zeigt, daß der Einbau eines Spacers zwischen Hapten und Aequorin den Erhalt der Photoproteinaktivität verbessert, desgleichen die Immunreaktivität und damit auch die Eichkurvendynamik sowie die Korrelation zum Enzymun®-Testformat. Die besten Ergebnisse wurden mit Spacern erhalten, die eine Kettenlänge von 2 bis 8 Atomen aufwiesen.

Beispiel 9

Vergleich von NHS-direktgekoppelten Konjugaten mit MH-SH-gekoppelten Konjugaten

Es wurden folgende Konjugate untersucht:

| Kopplungstyp | Kopplungsstöchiometrie | Einbaurate | %Restaktivität |
|---|---|---|---|
| NHS | 2:1 | 0.47 | 51 |
| MADOO-SH[a] | 2:1 | 0.51 | 17 |
| MH-SH[b] | 2:1 | 0.57 | 30 |

[a] = MADOO-Spacer (1-maleinimido-8-amino-3,6-dioxa-octyl-)
[b] = MH-Spacer (1-maleinimido-6-aminocaproyl-)

Dieses Experiment zeigt, daß bei einer innerhalb der Bestimmungsgenauigkeit gleichen Einbaurate die % Wiederfindung an Photoproteinaktivität bei Verwendung der NHS-Direktkopplung von Hapten-Aktivester an Aminogruppen von unmodifiziertem Aequorin klar besser ausfällt als bei Kopplung von MADOO- bzw. MH-aktiviertem Hapten und SH-aktiviertem Aequorin. In gleicher Weise ist die spezifische Aktivität in [counts/mg] bei direktgekoppelten Tracern höher als bei MADOO- bzw. MH-SH-gekoppelten Tracern.

**Patentansprüche**

1. Verfahren zum Nachweis eines Analyten in einer Probeflüssigkeit nach dem Prinzip des kompetitiven Immunoassay, **dadurch gekennzeichnet**, daß man

   (a) die Probeflüssigkeit in Gegenwart einer reaktiven Festphase mit (i) einem Konjugat aus mindestens einem mit dem Analyten immunologisch konkurrierenden Hapten und einem Ca-aktivierbaren Photoprotein und (ii) einem Rezeptor inkubiert, wobei das Hapten an eine Aminogruppe des Photoproteins gekoppelt ist, und der Rezeptor an die Festphase gebunden oder zur Bindung an die Festphase fähig ist und eine spezifische immunologische Reaktion mit dem Analyten und dem Hapten eingehen kann,
   (b) die Festphase von der Inkubationsflüssigkeit abtrennt und
   (c) das Vorhandensein oder/und die Menge des Analyten in der Probeflüssigkeit durch Lumineszenzmessung in der Festphase oder/und in der Inkubationsflüssigkeit bestimmt.

2. Verfahren nach Anspruch 1, **dadurch gekennzeichnet**, daß das Hapten über eine Carbonsäurefunktion an das Photoprotein gekoppelt ist.

3. Verfahren nach Anspruch 1 oder 2, **dadurch gekennzeichnet**, daß bei der Herstellung des Konjugats die Kopplung des Haptens an das Photoprotein in einem molaren Verhältnis von 1:1 bis 6:1 erfolgt.

4.  Verfahren nach einem der vorhergehenden Ansprüche,
    **dadurch gekennzeichnet**,
    daß das Hapten über einen Spacer an das Photoprotein gekoppelt ist.

5.  Verfahren nach einem der vorhergehenden Ansprüche,
    **dadurch gekennzeichnet**,
    daß die Inkubationsdauer in Schritt (a) maximal 45 Minuten beträgt.

6.  Verfahren nach einem der vorhergehenden Ansprüche,
    **dadurch gekennzeichnet**,
    daß die Inkubation in Schritt (a) als 1-Schritt-Simultaninkubation durchgeführt wird.

7.  Verfahren nach einem der vorhergehenden Ansprüche,
    **dadurch gekennzeichnet**,
    daß das Photoprotein im Konjugat eine Lumineszenzaktivität von mindestens 30% der Ausgangsaktivität besitzt.

8.  Verwendung eines Konjugats aus mindestens einem Hapten und einem Ca-aktivierbaren Photoprotein, worin das Hapten an eine Aminogruppe des Photoproteins gekoppelt ist, als Analytanalogon in einem kompetitiven Immunoassay.

9.  Reagenz zum Nachweis eines Analyten nach dem Prinzip des kompetitiven Immunoassay,
    **dadurch gekennzeichnet**,
    daß es ein Konjugat aus mindestens einem mit dem Analyten immunologisch konkurrierenden Hapten und einem Ca-aktivierbaren Photoprotein enthält, worin das Hapten an eine Aminogruppe des Photoproteins gekoppelt ist.

10. Reagenzienkit zum Nachweis eines Analyten nach dem Prinzip des kompetitiven Immunoassay, umfassend ein Reagenz nach Anspruch 9 und räumlich getrennt davon einen Rezeptor, der an eine Festphase gebunden oder zur Bindung an eine Festphase fähig ist und eine spezifische immunologische Reaktion mit dem Analyten und dem Hapten eingehen kann.

11. Konjugat aus mindestens einem Hapten und einem Ca-aktivierbaren Photoprotein, worin das Hapten über einen Spacer mit einer Kettenlänge von 2-8 Atomen an eine Aminogruppe des Photoproteins gekoppelt ist.